# EUROPÄISCHE PATENTSCHRIFT

(11) **EP 0 772 475 B1**
(45) Veröffentlichungstag und Bekanntmachung des Hinweises auf die Patenterteilung: **12.01.2005**
(21) Anmeldenummer: 95927637.9
(22) Anmeldetag: 28.07.1995
(51) Int. Cl.: A61N 1/39

(54) **VORRICHTUNG ZUR AUFNAHME VON FÜR HERZAKTIONEN CHARAKTERISTISCHEN SIGNALEN**
DEVICE FOR RECORDING CHARACTERISTIC SIGNALS OF CARDIAC ACTIVITY
DISPOSITIF D'ENREGISTREMENT DE SIGNAUX CARACTERISTIQUES DE L'ACTIVITE CARDIAQUE

(30) Priorität: 30.07.1994 DE 4427845
(43) Veröffentlichungstag der Anmeldung: 14.05.1997
(73) Patentinhaber: Biotronik GmbH & Co. KG, 12359 Berlin (DE)
(72) Erfinder: BOHEIM, Gustav, Lake Oswego, OR 97035 (US); WYBORNY, Paul, Lake Oswego, OR 97035 (US); DIGBY, Dennis, Lake Oswego, OR 97035 (US); THONG, Tran, Lake Oswego, OR 97035 (US); SCHALDACH, Max, D-91054 Erlangen (DE)
(74) Vertreter: Eisenführ, Speiser & Partner
(86) Internationale Anmeldenummer: PCT/DE1995/001030
(87) Internationale Veröffentlichungsnummer: WO 1996/004042

(56) Entgegenhaltungen:
- EP-A- 0 398 488
- EP-A- 0 402 508
- EP-A- 0 607 637

## Beschreibung

Die erfindung betrifft eine Vorrichtung zur gewinnung von Steuersignalen für einen kombinierten Herzschrittmacher/Defibrillator.

Implantierbare Herzschrittmacher und Defibrillatoren weisen eine mit einer intrakardialen Elektrode verbundene Eingangsstufe zur Aufnahme und Verstärkung von intrakardial über die Elektrode abgegriffenen Herzaktionspotentialen und eine Auswertungseinheit zu deren Auswertung und zur Ableitung von Steuersignalen zum Betrieb des Schrittmachers bzw. Defibrillators auf. Werden zur Steuerung sowohl Signale aus der Herzkammer (dem Ventrikel) als auch aus dem Vorhof (Atrium) benötigt, ist dort jeweils eine Elektrode vorgesehen, und die Eingangsstufen (ggf. auch Teile der Auswertungseinheit) bilden üblicherweise getrennte Kanäle, deren Verarbeitungscharakteristika (Empfindlichkeit bzw. Nachweisschwelle, Filter- und Verstärkungsparameter) getrennt einstellbar sind.

Bei automatischen Defibrillatoren und Schrittmachern mit Doppelfunktion, die erforderlichenfalls als Defibrillator arbeiten, müssen die Eingangsstufen ohne irgendeinen Bedienereingriff sowohl die sich bei normaler Herztätigkeit (Sinusrhythmus) einstellenden Herzaktionspotentiale bzw. - signale als auch Signale erfassen und vorverarbeiten, wie sie bei den verschiedenen Arrhythmiezuständen des Herzens auftreten.

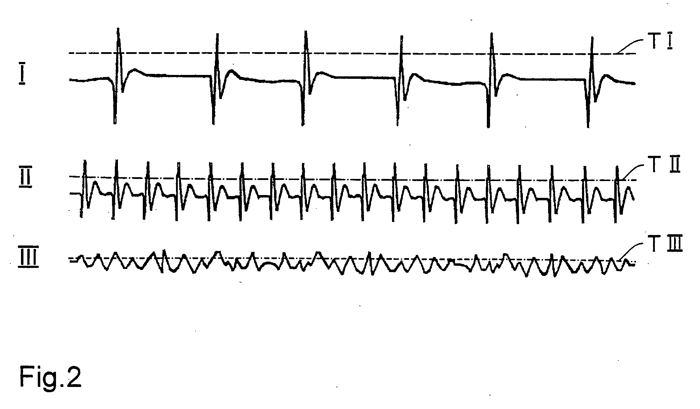

Die Signalamplituden der intrakardial gewonnenen Signale, die verschiedene Herzrhythmus-Zustände charakterisieren, unterscheiden sich erheblich voneinander, wie in Fig. 2 zu erkennen ist. Kurve I verdeutlicht hier einen typischen Sinusrhythmus bei normaler Herzfunktion, Kurve II das Elektrogramm einer ventrikulären Tachykardie und Kurve III dasjenige eines Kammerflimmerns (ventrikulärer Fibrillation). Mit einer gestrichelten Linie (in Kurve I), einer strichpunktierten Linie (in Kurve II) bzw. einer doppeltstrichpunktierten Linie (in Kurve III) ist jeweils eine angemessene Nachweisschwelle TI, TII bzw. TIII bezeichnet.

Es ist - etwa aus EP 0 349 130 A1 - bekannt, die Eingangsstufe eines Schrittmachers mit einer automatischen Verstärkungsregelung (AGC = automatic gain control) auszuführen. Diese dient hier im Zusammenwirken mit einer Bandpaßfilterung zur Verbesserung des Signal-/Rausch-Verhältnisses.

Es ist ferner aus US 4 184 493 A1 bekannt, eine automatische Verstärkungsregelung in einem automatischen, implantierbaren Defibrillator vorzusehen. Diese bewirkt hier zusammen mit einer Hochpaßfilterung eine weitgehende Unterdrückung von S- und T-Anteilen des Elektrogramms und verhindert damit eine auf die Erfassung dieser Signalanteile gestützte mögliche Fehlinterpretation eines "normalen" Elektrogramms als Kammerflimmern.

Eine weitergehende Verwendung im Zusammenhang mit dem Nachweis von Signalanteilen niedriger Amplitude und damit einer ventrikulären Fibrillation erfährt die AGC in einem implantierbaren Kardioverter/Schrittmacher nach DE 37 39 014 A1.

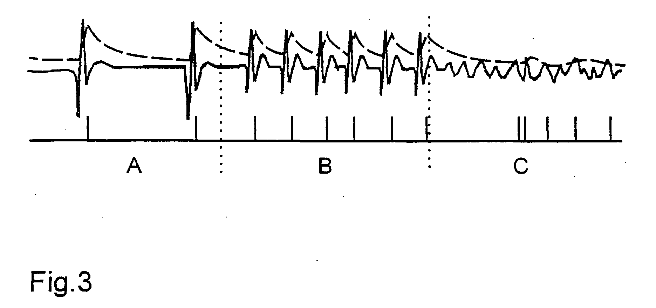

Eine solche Funktion der AGC ist in Fig. 3 verdeutlicht, wo die durchgezogene Kurve ein Elektrogramm darstellt, in dessen linkem Abschnitt (Bereich A) Sinusrhythmus, in dessen mittlerem Abschnitt (Bereich B) eine ventrikuläre Tachykardie und in dessen rechtem Abschnitt (Bereich C) Kammerflimmern zu erkennen ist. Die obere, gestrichelte Linie stellt die effektive Nachweisschwelle dar, wie sie durch die AGC eingestellt wird, und im unteren Teil der Figur sind die beim angegebenen zeitlichen Verlauf der Nachweisschwelle nachgewiesenen Ereignisse dargestellt.

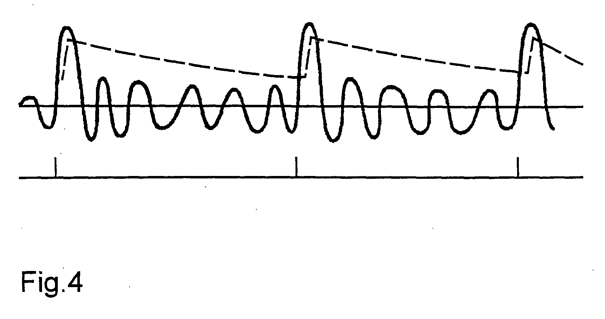

Bestimmte Erscheinungsbilder ventrikulärer Fibrillation sind im intrakardialen Elektrogramm durch das Auftreten eines relativ niederfrequenten Signalmusters mit vergleichsweise hoher Amplitude in Überlagerung zu den wesentlich höherfrequenten und schwächeren Fibrillationssignalen gekennzeichnet, vgl. dazu etwa US 4 523 595, speziell Fig. E12 und E13. Ein solches Elektrogramm zeigt (schematisch) die analog zu Fig. 3 aufgebaute Fig. 4. Wie diese Figur verdeutlicht, verhindert die AGC mit dem relativ langsamen Anstieg der Verstärkung und dem entsprechend langsamen Absinken der Nachweisschwelle nach jedem, jeweils die Schwelle erhöhenden, Signal des überlagerten Signalmusters eine Erkennung der die Fibrillation kennzeichnenden Signale. Damit kann der Defibrillator nicht in Funktion treten, obwohl der Einsatzfall vorliegt.

Die automatische verstärkungsregelung (AGC) bringt bei einem automatischen Defibrillator also u.U. schwerwiegende Funktionsmängel mit sich - ganz abgesehen davon, daß ihre Realisierung für stark differenzierte Signalbilder in der Art kardialer Elektrogramme nicht einfach und recht kostenaufwendig ist.

Der Erfindung liegt daher die Aufgabe zugrunde, eine Vorrichtung der eingangs genannten Gattung, mit denen eine zuverlässige Erfassung und Unterscheidung verschiedener Herzzustände aufgrund eines, intrakardialen Elektrogramms mit vertretbarem Aufwand möglich ist, sowie einen sich dieser Vorrichtung bedienenden kombinierten Herzschrittmacher / Defibrillator anzugeben.

Diese Aufgabe wird durch eine Vorrichtung mit den Merkmalen des Anspruchs 1 gelöst.

Die Erfindung schließt den Gedanken ein, eine Signalvorverarbeitung intrakardial aufgenommener Herzsignale unter Anwendung zweier verschiedener permanent wirksamer, während der Messung unverändert bleibender Nachweisschwellen vorzunehmen, von denen eine speziell auf die Erfassung von Fibrillations-Signalen zugeschnitten ist. Damit wird die bei der automatischen Verstärkungsregelung unvermeidliche Gefahr des "Übersehens" von Signalen kleiner Amplitude, die ein Herzflimmern signalisieren, bei gleichzeitigem Auftreten von Signalen mit wesentlich größerer Amplitude beseitigt, und es kann zugleich durch den Wegfall der aufwendigen AGC der Aufbau der Erfassungsschaltung vereinfacht werden.

Die zugrundegelegten Signale können insbesondere unter Verwendung mindestens einer intrakardial angeordneten Sensing-Elektrode - wobei sie den zeitlichen Verlauf eines Herzaktionspotentials am Aufnahmeort darstellen - oder ggfs. auch mittels eines oder mehrerer intrakardial angeordneter Druckwandler - wobei ein intrakardiale zeitliche Druckschwankungen reflektierendes elektrisches Signalspektrum vorliegt - aufgenomen werde. Es sind aber auch andere Aufnehmer einsetzbar, die grundsätzlich das Auftreten von Fibrillationsereignissen anzeigende Signale liefern können.

Die Realisierung der verschiedenen Nachweisschwellen erfolgt in jeweils einer gesonderten Eingangsstufe mit an sich bekanntem Aufbau, wobei das in Abhängigkeit von der Zeit aufgenommene Signalspektrum einer Auswertung des Schwellwert-Vergleichsergebnisses zur Gewinnung einer Aussage hinsichtlich des Auftretens sinusartiger Herzaktionen oder von Herzflimmern (Fibrillationen) unterzogen und letztlich ein das Auswertungsergebnis charakterisierendes Steuersignal erzeugt wird, das zur Steuerung eines Schrittmachers / Defibrillators dienen kann.

Die Eingangstufe mit der niedrigen Nachweisschwelle umfaßt einen Breitband verstärker mit hohem Verstärkungsfaktor, wobei eine Pegelbegrenzerschaltung zur Abschneidung von oberhalb einer vorbestimmten, oberhalb des Pegels der niedrigeren Nachweisschwelle gewählten, Grenze liegenden Signalanteilen - insbesondere auch von Reizimpuls-Artefakten - die Übertragungseigenschaften für die schwachen Fibrillations-Signale verbessert. Eine weitere Verbesserung ist diesbezüglich durch eine Austast- oder Blankingschaltung Ausblendung der Signalanteile während einer vorbestimmten Zeitspanne nach dem Auftreten eines oberhalb der vorbestimmten Grenze liegenden Signalanteils erzielbar.

Eine Digitalisierung ermöglicht in kostengünstiger Weise den Einsatz eines A/D-Wandlers mit geringer Genauigkeit bzw. Verarbeitungsbreite und anschließend den Einsatz eines digitalen Komparators zur Schwellwertdiskriminierung und eine einfache digitale Analyse in einem Mikroprozessor oder einer integrierten "customer-circuit"-Auswertungsschaltung. Weiter ermöglicht es auf einfache Weise, die Signale für eine spätere anderweitige Analyse zwischenzuspeichern

Die Einstellung der niedrigeren Nachweisschwelle kann in günstiger Weise in Abhängigkeit vom Ergebnis einer Auswertung der maximalen oder einer mittleren Amplitude oder der Signalenergie des keiner Abschneidung unterzogenen Anteils des Gesamt-Signalspektrums während einer initialen Messung vorgenommen werden.

Um die schwachen Fibrillationssignale mit hinreichender Sicherheit vom Rauschen zu unterscheiden und somit u.U. gefahrvolle Fehlalarme eines Defibrillators zu verhindern, ist eine Unterscheidung der der zweiten Eingangsstufe zugeführten oder in ihr vorverarbeiteten Signale von Rauschen aufgrund einer Amplituden- und/oder einer Frequenzdiskriminierung praktisch zweckmäßig. Diese wird zweckmäßig anhand digitalisierter Signale durchgeführt. Im Falle einer Amplitudendiskriminierung wird eine Grenze vorgegeben, oberhalb derer liegende Signale als signifikant und unterhalb derer. liegende Signale als Rauschen klassifiziert werden. Die (niedrige) Schwelle wird dann beispielsweise auf 75% des Spitzenpegels der als signifikant beurteilten Signale gesetzt.

Die Auswertung kann auch eine zeitliche Mittelung bezüglich der mit der niedrigen Schwelle vorverarbeiteten Nachweissignalfolge über eine vorbestimmte Anzahl von Signalen oder eine vorbestimmte Zeitperiode zur Rauschunterdrückung und die Bestimmung einer mittleren Rate dieser Signale aufweisen, wobei in Abhängigkeit von der mittleren Rate ein das Vorliegen oder Nicht-vorliegen von Fibrillationen kennzeichnendes Signal ausgegeben wird.

Weiterhin ist es möglich, dass eine Bestimmung der Signal-Spitzenwerte oder eines Signal-Mittelwertes oder der mittleren Signalleistung bzw. des quadratischen Mittels der Signalamplitude der über der zweiten Nachweisschwelle liegenden Signale vorgenommen wird und in Abhängigkeit von der mittleren Rate und dem Signal-Spitzenwert, dem Signal-Mittelwert oder dem quadratischen Mittel ein das Vorliegen oder Nicht-Vorliegen von Fibrillationen kennzeichnendes Signal ausgegeben wird. Dies setzt das Vorliegen von typischen bzw. Vergleichs-Signalbildern voraus, die dem Kardiologen jedoch allgemein bekannt sind oder - patientenspezifisch - etwa bei provozierten Fibrillationen ermittelt werden können.

Eine besonders vorteilhafte Nutzung des Zwei-Schwellen-Prinzips gemäß der Erfindung gestaltet sich derart, daß die Auswertung eine zeitliche Mittelung bezüglich der mit der höheren, auf aus sinusartigen Herzereignissen herrührenden Signale abgestimmten, Schwelle registrierten Nachweissignalfolge über eine vorbestimmte Anzahl von Signalen oder eine vorbestimmte Zeitperiode zur Bestimmung einer mittleren Rate dieser Signale umfaßt, aus der mittleren Rate ein Zeitfenster (Escape-Intervall, etwa spezifisch für Bradykardie) bestimmt wird und in Abhängigkeit vom Auftreten oder Nicht-Auftreten von über der ersten Nachweisschwelle sowie von über der zweiten Nachweisschwelle liegenden Signalanteilen innerhalb des Zeitfensters ein die Art der aktuellen Herztätigkeit insgesamt (etwa auch eine Bradykardie) kennzeichnendes Signal ausgegeben wird.

Bei der erfindungsgemäßen Vorrichtung kann jeder Eingangsstufe eine separate Auswertungseinheit mit einem Steuersignalausgang zur Ausgabe eines die jeweiligen Auswertungsergebnisse charakterisierenden Steuersignals zugeordnet. sein. Es ist aber auch eine abschnittsweise gemeinsame oder verknüpfte Auswertung - etwa im Sinne des vorigen Absatzes - möglich.

Weiterhin kann die Eingangstufe mit der niedrigen Schwelle zweckmäßigerweise einen A/D-Wandler zur Digitalisierung der aufgenommenen oder der bereits vorverarbeiteten Signale aufweisen, und die Vergleichereinheit zur Schwellwertdiskriminierung kann dann als digitaler Vergleicher ausgeführt sein. Der Eingang des A/D-Wandlers ist mit dem Ausgang der Pegelbegrenzerschaltung verbunden und der A/D-Wandler ist ein solcher mit relativ geringer Verarbeitungsbreite. Weiterhin kann ein Signalspeicher vorgesehen sein, dessen Dateneingang mit dem Ausgang des A/D-Wandlers und dessen Datenausgang mit einer internen oder externen Auswertungseinheit verbunden werden kann.

Der ersten und zweiten Eingangsstufe können Zeitgebermittel (Timer) zur Bestimmung von Auswertungszeitintervallen zugeordnet sein und sie jeweils eine Ratenbestimmungsschaltung zur Bestimmung einer mittleren Rate der über der jeweiligen Nachweisschwelle liegenden Signalanteile aufweisen. Alternativ oder zusätzlich können Mittel zur Amplitudendiskriminierung und ggf. Amplituden-Mittelwertbildung sowie Mittel zur Einstellung der zweiten Nachweisschwelle in Abhängigkeit vom Ergebnis einer Auswertung der maximalen oder einer mittleren Amplitude des keiner Abschneidung unterzogenen Anteils des Gesamt-Signalspektrums während einer initialen Meßperiode vorgesehen sein.

Die Mittel zur Amplitudendiskriminierung werden - falls eine digitale Signalverarbeitung erfolgt - zweckmäßigerweise dem A/D-Wandler nachgeschaltet sein.

Die erfindungsgemäße Vorrichtung findet Verwendung bei einem automatischen Defibrillator, der außerdem als Bedarfssschrittmacher fungiert.

Vorteilhafte Weiterbildungen der Erfindung sind in den Unteransprüchen gekennzeichnet bzw. werden nachstehend zusammen mit der Beschreibung der bevorzugten Ausführung der Erfindung anhand der Figuren näher dargestellt. Es zeigen:
Figur 1 ein stark vereinfachtes Blockschaltbild eines Zweikammer-Bedarfsschrittmachers mit Standby-Defibrillator, in dem eine Ausführungsform der Erfindung realisiert ist,
Figur 2 eine Darstellung von intrakardial aufgenommenen Elektrogrammen (EKG-Signalen) verschiedener Herzaktionen,
Figur 3 eine schematisierte Darstellung eines Elektrogramms verschiedener, zeitlich aufeinanderfolgender Herzaktionen unter Kennzeichnung des zeitlichen Verlaufs der Nachweisschwelle und der nachgewiesenen Signale nach dem Stand der Technik (mit AGC),
Figur 4 eine schematische Darstellung eines spezielllen Elektrogramms' unter Kennzeichnung des zeitlichen Verlaufs der Nachweisschwelle und der nachgewiesenen Signale nach dem Stand der Technik (mit AGC),
Figur 5 eine schematische Darstellung des Elektrogramms nach Fig. 4 unter Kennzeichnung des zeitlichen Verlaufs der Nachweisschwellen und der jeweils nachgewiesenen Signale bei einer Ausführungsform der Erfindung,
Figur 6 eine schematische Darstellung des Elektrogramms nach Fig. 4 und 5 bei höherer Verstärkung, mit Spitzenabschneidung und Austastung von signalabschnitten bei einer weiteren Ausführungsform der Erfindung und
Figur 7. ein stark vereinfachtes Blockschaltbild einer Leseschaltung in einer Ausführungsform der Erfindung.

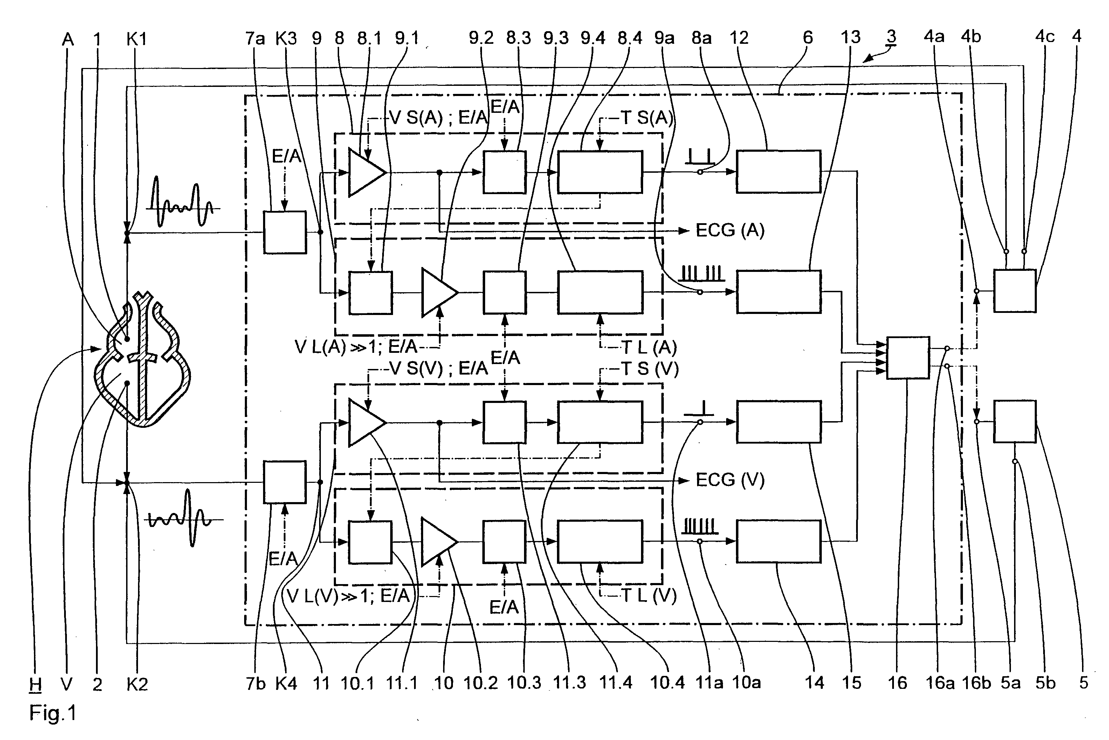

In Fig. 1 ist in stark vereinfachter Darstellung - insbesondere unter Fortlassung der Komponenten zur Stromversorgung, Programmierung etc. - ein mit Elektroden 1 im Atrium A und 2 im Ventrikel V eines Herzens H verbundener Zweikammerschrittmacher 3 mit einer Schrittmacherimpulseinheit 4 und einer integrierten Defibrillator-Entladungsstufe 5 gezeigt.

Die Schrittmacherimpulseinheit 4 weist einen Steuereingang 4a und zwei getrennte Impulsausgänge 4b und 4c für atriale bzw. ventrikuläre Stimulationsimpulse auf. Der Ausgang 4b ist über einen Knoten K1 mit der atrialen Elektrode 1 und der Ausgang 4c über einen Knoten K2 mit der ventrikulären Elektrode 2 verbunden. Die Defibrillator-Entladungsstufe 5 weist einen Steuereingang 5a und einen Impulsausgang 5b für Kardiovertierungsimpulse auf, der ebenfalls über den Knoten K2 mit der ventrikulären Elektrode 2 verbunden ist. (In ihrer Funktion als Defibrillationselektrode ist die Elektrode 2 hier lediglich schematisch gezeigt; es können daneben zur Kardiovertierung weitere intrakardiale oder subkutane Elektroden vorgesehen sein.)

Die Elektroden 1 und 2 dienen außer als Reizelektroden als Signalaufnehmer für atrial bzw. ventrikulär aufgenommene Elektrogramme (intrakardiale EKG-Signale). Sie sind daher über die Knoten K1 bzw. K2 auch mit einer Lese- und Auswertungsschaltung 6 des Schrittmachers/Defibrillators 3 verbunden. Ihre Ausgangssignale gelangen über ein-/ausschaltbare (etwa unmittelbar durch abgegebene Stimulations- oder Defibrillationsimpulse getriggerte) Austast- bzw. "Blanking"-Stufen 7a und 7b zum'Schutz vor Übersteuerung durch Stimulationsimpulse auf Knoten K3 bzw. K4, wo sich der Signalweg für das atriale und ventrikuläre Signal jeweils verzweigt.

Vom Knoten K3 aus wird das atriale Signal zwei getrennten Eingangsstufen 8 und 9 zugeführt, und das ventrikuläre Signal wird vom Knoten K4 aus zwei getrennten Eingangsstufen 10 und 11 zugeführt.

Der weitere Signalweg ist für beide Signale im Prinzipabgesehen von spezifischen Einstellungen der Baugruppengleich, so daß nachfolgend nur der Signalweg für das atrial aufgenommene Signal beschrieben wird. Den einzelnen Baugruppen der Eingangsstufen 8 und 9 entsprechen in den Eingangsstufen 10 und 11 die Baugruppen mit der analogen Numerierung, also der Baugruppen 8.1 und 9.1 die Baugruppen 10.1 und 11.1 usw.

Die erste Eingangsstufe B für das atriale Signal weist eidurch entsprechende, mit "E/A" bzw. "VS(A)" bezeichnete Steuersignale ein-/ausschaltbaren und in seiner Verstärkung einstellbaren Leseverstärker 8.1 und die Eingangsstufe 9 einen Leseverstärker 9.2 auf. Letzterer ist breitbandig ausgelegt, ebenfalls über ein Signal "E/A" wahlweise ausschaltbar und hat einen relativ großen, über ein Steuersignal "VL(A)" einstellbaren Verstärkungsfaktor.

Innerhalb der ersten Eingangsstufe B gelangt das verstärkte Signal vom Leseverstärker 8.1 zu einer - ebenfalls über ein Signal "E/A" ein-/ausschaltbaren - Filterstufe 8.3 und von dieser zu einer Schwellwertdetektorschaltung 8.4 mit über ein Steuersignal "TS(A)" einstellbarer Nachweisschwelle, die im Bereich der üblichen Nachweisschwellen von Schrittmachereingangsschaltungen für sinsusartige Herzereignisse (ohne AGC) liegt. Außerdem ist zwischen dem Leseverstärker 8.1 und der Filterstufe 8.3 ein Abgriffspunkt für ein ungefiltertes intrakardiales EKG-Signal "ECG(A)" vorgesehen.

Innerhalb der zweiten Eingangsstufe 9 gelangt das Eingangssignal zunächst zu einer integrierten Pegelbegrenzerstufe und Austast- bzw. Blanking-Schaltung 9.1, die über ein Steuersignal von der Schwellwertdetektorschaltung 8.4 der ersten Eingangsstufe 8 aktiviert werden kann und Übersteuerungen in diesem Signalweg verhindert. Vom Ausgang dieser Stufe gelangt es zum Leseverstärker 9.2 und als breitbandig verstärktes Signal weiter zu einer - wiederum über ein Signal "E/A" ein-/ausschaltbaren - Filterstufe 9.3. Von dieser gelangt es schließlich zu einer Schwellwertdetektorschaltung 9.4 mit über ein Steuersignal "TL(A)" einstellbarer Nachweisschwelle, die unterhalb der Schwelle der ersten Schwellwertdetektorschaltung 8.4 und der üblichen Nachweisschwellen von Schrittmachereingangsschaltungen (ohne AGC) liegt.

Die Baugruppen 8.1, 8.3 und 8.4 bilden die erste und die Baugruppen 9.1, 9.2, 9.3 und 9.4 die zweite Eingangsschaltung für das atriale Meßsignal.

In den Schwellwertdetektorstufen 8.4 bzw. 9.4 wird das Eingangssignal nach Maßgabe der eingestellten Nachweisschwelle jeweils auf an sich bekannte Weise in eine Folge von Einzelimpulsen umgewandelt. Die Impulsfolgen werden über die Signalausgänge 8a bzw. 9a zunächst getrennten Auswertungsstufen 12 bzw. 13 zugeführt, in denen sie zur Klassifizierung bzw. Identifizierung der durch die atriale Messung nachgewiesenenen Herzereignissen genutzt werden.

Die in den Stufen 10.1 bis 10.4 bzw. 11.1 bis 11.4 analog zur vorstehenden Beschreibung verarbeiteten ventrikulären Signale werden in Auswertungsstufen 14 bzw. 15 auf ähnliche Weise analysiert, und alle Auswertungs- bzw. Zwischenergebnisse werden anschließend einer zentralen Verarbeitungs- und Steuereinheit 16 zugeführt, die schließlich an Ausgängen '16a und 16b Steuersignale zum Betrieb der Schrittmacherimpulseinheit 4 bzw. der Defibrillatorstufe 5 bereitstellt.

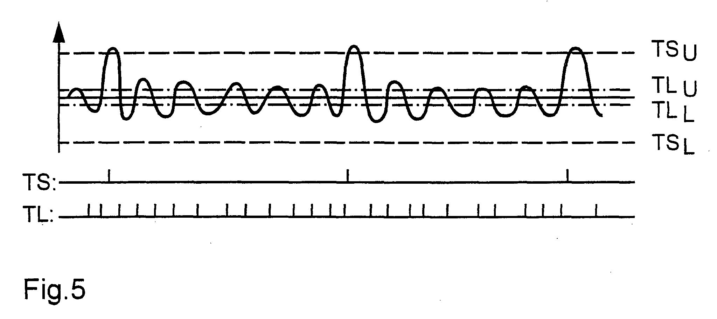

Die Funktionsweise der in Fig. 1 gezeigten Anordnung wird unter Bezugnahme auf Fig. 5 erläutert, die eine schematische Darstellung eines (atrial oder ventrikulär) aufgenommenen Elektrogramms unter Kennzeichnung des zeitlichen Verlaufs der Nachweisschwellen der beiden der Elektrode 1 oder 2 zugeordneten Eingangsstufen 8 und 9 oder 10 und 11 sowie unter Angabe von deren Ausgangssignalfolgen ist.

Bei Fig. 5 wird - abweichend von in der Praxis üblicherweise vorzunehmenden Einstellungen - vereinfachend angenommen, daß beide Leseverstärker denselben Verstärkungsfaktor aufweisen und keine Pegelabschneidung, Austastung oder unterschiedliche Filterung in beiden Signalwegen erfolgt ist. Dann liegt am Eingang der Schwellwertdetektoren 8.4 und 9.4 (oder 10.4 und 11.4) das gleiche Signalspektrum an. Bei Diskriminierung mit den in Fig. 5 angegebenen Nachweisschwellen TS_{U}, TS_{L} und TL_{U}, TL_{L} ergeben sich die im unteren Teil der Figur - mit gleicher Zeitskala wie im oberen Teil - angegebenen Nachweissignalfolgen "TS" bzw. "TL".

Ein Vergleich mit der eingangs der Beschreibung erläuterten Fig. 4, die das gleiche Elektrogramm zeigt, verdeutlicht den durch die Anwendung zweier zeitkonstanter Nachweisschwellen erzielten Gewinn:

Während beim üblichen verfahren der Aufbereitung der Eingangssignale in einer Eingangsstufe mit AGC die unterhalb des größeren Signals liegenden Signalanteile, die ein Herzflimmern (Fibrillationen) anzeigen, nicht nachgewiesen werden können, gelingt dies mittels der oben beschriebenenen Vorrichtung ohne weiteres. Darüber hinaus gelingt - was bei Anwendung nur einer, niedrigen Schwelle nicht der Fall wäre - eine Vor-Klassifizierung der Signale, im gezeigten Elektrogramm die Trennung zwischen den Fibrillations- und den auf eine überlagerte reguläre Herzaktion hinweisenden Signalen. Dies ermöglicht eine exakte Beurteilung des Zustands des Herzens in den folgenden Auswertungsstufen und die korrekte Steuerung des Schrittmachers oder des Defibrillators.

Durch den Einsatz der Pegelbegrenzer- und Austastschaltungen 9.2 und 11.2 wird das Übertragungsverhalten in den Signalwegen mit der niedrigen Nachweisschwelle noch verbessert. Durch deren Aktivierung, während durch Eingangssignale mit hohem Pegel die Standard-Nachweisschwelle der Eingangsstufe 8 überschritten ist, wird eine hohe Verstärkung in der Eingangsstufe 9 ermöglicht, was einer niedrigen effektiven Schwelle gleichkommt. Dies kann derart genutzt werden, daß die in der Diskriminatorschaltung 9.4 eingestellte Detektor-Schwelle im üblichen Bereich liegen kann und die Eingangsstufe 9 dennoch eine niedrige effektive Nachweisschwelle hat.

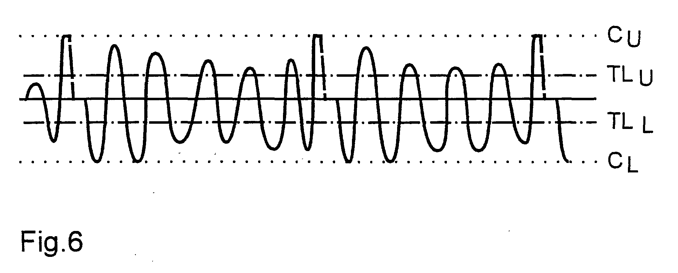

Ein mit Pegelbegrenzung und Austastung verarbeitetes Elektrogramm entsprechend Fig. 4 und 5 ist in Fig. 6 dargestellt. Die obere und untere Nachweissschwelle TL_{U} bzw. TL_{L} entsprechen Fig. 5; die. in Fig. 6 angenommene hohe Verstärkung bedingt jedoch einen anderen Maßstab der Ordinate gegenüber Fig. 5. Die Bereiche des Elektrogramms, wo eine Pegelbegrenzung auf einen voreingestellten Pegelwert C_{U} eingesetzt hat, sind in Figur 6 erkennbar. In der Figur ist zu erkennen, daß sich jeweils ein Austastbereich anschließt.

Die Pegelbegrenzung erleichtert im übrigen eine Digitalisierung und digitale Weiterverarbeitung der Signale, da sich damit die Anzahl der erforderlichen Quantisierungsstufen und die Verarbeitungsbreite verringert. Insbesondere können die Schwellwertdetektionsstufen dann digitale Komparatoren aufweisen und die Auswertungen. in einer Mikroprozessor-Konfiguration vorgenommen werden.

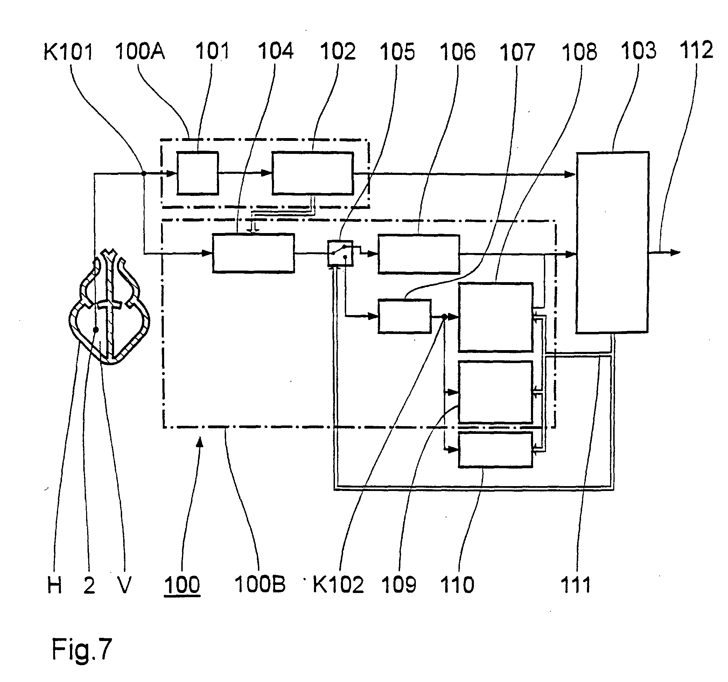

Eine entsprechende Lese- und Auswertungsschaltung 100 als weitere Ausführungsform der erfindungsgemäßen Vorrichtung ist schematisch in Fig. 7 dargestellt.

Ein über eine Sensing-Elektrode 2 im Ventrikel V eines Herzens H aufgenommenes Herzaktionspotential gelangt über einen Knoten K101 einerseits zu einem herkömmlichen Leseverstärker 101 und von diesem zu einer ersten integrierten Schwellwertdetektor- und Ratenbestimmungsschaltung 102, von der im Ergebnis der Schwellwertdiskriminierung und einer Ratenbestimmung der Signalanteile mit großer Amplitude gewonnene Ausgangssignale an einen Mikroprozessor 103 übergeben werden.

Andererseits gelangt das Eingangssignal zu einer integrierten Blanking- und Breitbandverstärkerschaltung 104 mit hoher Verstärkung, deren Blanking-Verhalten einer von der Stufe 102 ausgehenden Steuerung unterliegt.

Das verstärkte Signal wird durch eine Schalteinheit 105 wahlweise einer (analogen) Schwellwertdetektor- und Ratenbestimmungsschaltung 106, von der im Ergebnis der Schwellwertdiskriminierung und einer Ratenbestimmung der Signalanteile mit kleiner Amplitude gewonnene Ausgangssignale an den Mikroprozessor 103 übergeben werden, oder zunächst einem A/D-Wandler 107 zugeführt.

Der Ausgang des A/D-Wandlers 107 ist über einen Knoten K102 mit den Eingängen einer digitalen Schwellwertdetektor- und Ratenbestimmungsschaltung 108, eines digitalen Signalprozessors 109 und eines digitalen EKG-Speichers 110 verbunden, die sämtlich über einen Bus 111 mit dem Mikroprozessor 103 verknüpft sind. Der Ausgang der Stufe 108 ist zudem über eine herkömmliche Signalleitung, über die (alternativ zu Ergebnissen der analogen Signalverarbeitung in Stufe 106) die Ergebnisse der digitalen Vararbeitung in Stufe 108 übergeben werden, mit dem Mikroprozessor verbunden.

Der Mikroprozessor 103 stellt Signale 112 für nachfolgende Verarbeitungs- und/oder Steuerstufen oder eine Ausgabe nach außen (etwa für eine externe EKG-Auswertung) bereit.

Die Baugruppen 101 und 102 bilden eine erste Eingangsstufe 100A mit Standard-Schwelle und die Baugruppen 104 bis 109 eine zweite Eingangsstufe 100B mit niedriger Schwelle.

Die Funktionsweise dieser Schaltung ist, soweit sie sich von der in Fig. 1 gezeigten unterscheidet, wie folgt:

In der ersten Schwellwertdetektor- und Ratenbestimmungsstufe 102 werden zunächst die Signalanteile mit hohem Pegel registriert, und deren mittlere Rate wird bestimmt. Im Vergleich mit gespeicherten Werten kann daraus (im Zusammenwirken mit der Mikroprozessor-Konfiguration 103, die auch einen entsprechenden Datenspeicher einschließt) zunächst auf das Vorliegen eines normalen Sinusrhythmus, einer Tachykardie oder eines möglichen Herzflimmerns geschlossen werden.

In der zweiten (analogen) Schwellwertdetektor- und Ratenbestimmungsschaltung 106 wird entsprechend - unter Einschluß einer Akkumulation bzw. Mittelung - die Rate der Signale mit niedrigem Pegel bestimmt und daraus (wiederum im Zusammenwirken mit dem Mikroprozessor und einem Datenspeicher) auf das Vorliegen von Fibrillationen geschlossen. Werden solche festgestellt, wird das auf dem verarbeitungsweg mit hoher Schwelle erhaltene Ergebnis ignoriert, das auf dem Weg mit niedriger Schwelle erhaltene Ergebnis unter Verkürzung des Mittelungs-Zeitintervalls verifiziert und - falls es sich bestätigt - eine Defibrillation eingeleitet.

Mit der dritten (digitalen) Schwellwertdetektions- und Ratenbestimmungschaltung 108 wird ähnlich vorgegangen, wobei zusätzlich etwa die Signalspitzen ausgewertet und zur Entscheidung über das Vorliegen von Herzflimmern mit herangezogen werden..

Dieser Verarbeitungsweg eröffnet weiterhin auf einfache Weise die Möglichkeit, das vorliegen einer Bradykardie zu verifizieren, indem ein Zeitfenster (Bradykardie-Escape-Intervall) vorgegeben wird, auf das alle Auswertungen bezogen werden. Tritt innnerhalb dieses Zeitfensters kein Signal mit hoher Amplitude auf und ist der Signal-Spitzenwert der Signale mit niedriger Amplitude im wesentlichen gleich dem mittleren Signalpegel oder liegt er unterhalb eines vorgegebenen Limits - das vorteilhaft gleich der niedrigen Schwelle gewählt wird - , so werden keine Fibrillationen, sondern nur Rauschen detektiert. Somit kann auf das Vorliegen einer Bradykardie geschlossen und die entsprechende Schrittmachertherapie eingeleitet werden.

Bei der Anordnung nach Fig. 7 können einige oder ggfs. auch alle die Signalverarbeitung betreffenden Funktionen des Mikroprozessors von einem kundenspezifischen ("customer-circuit") Verarbeitungs-Schaltkreis übernommen werden.

Die Einstellung der niedrigen Nachweisschwelle kann bei beiden Anordnungen nach Fig. 1 oder Fig. 7 aufgrund einer Messung des nicht begrenzten Signalpegels oder des Amplituden-Mittelwertes oder des quadratischen Mittels erfolgen, wobei ggf. im Sättigungsbereich liegende Signale und gewisse Signalanteile in der Umgebung der Sättigungen auszuklammern sind.

Wird die Schwelle selbst eingestellt, so braucht der Verstärkungsfaktor nicht verändert zu werden, was bei digitaler Schwellwertverarbeitung und einem Breitbandverstärker mit großem Verstärkungsfaktor von erheblichem Vorteil sein kann.

Die Erfindung beschränkt sich in ihrer Ausführung nicht auf das vorstehend angegebene bevorzugte Ausführungsbeispiel. Vielmehr ist eine Anzahl von Varianten denkbar, welche von der dargestellten Lösung auch bei grundsätzlich .anders gearteten Ausführungen Gebrauch macht.

## Patentansprüche

1. Vorrichtung zur Gewinnung von Steuersignalen für einem kombinierten Herzschrittmacher/Defibrillator durch Aufnahme von für Herzaktionen charakteristischen Signalen im Atrium (A) und/oder Ventrikel (V) eines Herzens (H)
und deren Auswertung
zur Gewinnung einer Aussage hinsichtlich des Auftretens auf normalem Sinusrhythmus beruhender Herzaktionen oder von Fibrillationen
mit
jeweils mindestens einem intrakardialen Signalaufnehmer (1, 2) im Atrium (A) und/oder Ventrikel (V) zur Aufnahme eines Signalspektrums in Abhängigkeit von der Zeit,
einer mit dem Signalaufnehmer zum Zuführen des über jeden Signalaufnehmer (1, 2) aufgenommenen Signalspektrums verbundenen Lese- und Auswertungsschaltung (6; 100) mit Schwellwertcharakteristik,
wobei in der Lese- und Auswertungsschaltung (6; 100)
jeder Signalaufnehmer (1, 2) zum Zuführen des jeweiligen Signalspektrums und dessen Aufbereitung verbunden ist umfassend
eine erste Eingangsstufe (8, 11) mit einem ersten Leseverstärker (8.1, 11.1) mit einem ersten einstellbaren, aber nach einer vorgenommenen Einstellung zeitlich konstantem Verstärkungsfaktor (VS(A); VS(V)) sowie mit
einer ersten Schwellwertdetektorschaltung (8.4, 11.4) mit einer ersten einstellbaren, aber nach einer vorgenommen Einstellung zeitlich konstanten Nachweisschwelle (TS(A), TS(V)), wobei der erste Lese verstärker (8.1, 11.1) den Verstärkungsfaktor bestimmt, mit dem das aufgenommene Signalspektrum bis zu dem Eingang der ersten Schwellwertdetektorschaltung verstärkt wird, und der erste Verstärkungsfaktor (VS(A), VS(V) zuzammen mit der ersten Nachweisschwelle (TS(A), TS(V)) auf die Signalamplitude von sich bei normalem Sinusrhythmus einstellenden Herzsignalen abgestimmt ist, und
eine zweite Eingangsstufe (9, 10) mit einem zweiten Leseverstärker (9.2, 10.2) mit einem zweiten einstellbaren, aber nach einer vorgenommenen Einstellung zeitlich konstantem Verstärkungsfaktor (VL(A); VL(V)) sowie mit
einer zweiten Schwellwertdetektorschaltung (9.4, 10.4) mit einer zweiten einstellbaren, aber nach einer vorgenommen Einstellung zeitlich konskanten Nachweisschwelle (TL(A), TL(V)), wobei der zweite Leseverstärker (9.2, 10.2) den Verstärkungsfaktor bestimmt, mit dem das aufgenommene Signalspektrum bis zu dem Eingang der zweiten Schwellwert detektorschaltung verstärkt wird, und der zweite Verstärkungsfaktor (VL(A), VL(V) zuzammen mit der zweiten Nachweisschwelle (TL(A), TL(V)) auf die Signalamplitude von Fibrillationsereignissen abgestimmt ist,
wobei die Schwellwertdetektorschaltungen (8.4, 9.4, 10.4, 11.4) jeweils für einen getrennten Vergleich der Signale des Signalspektrums mit der ersten und zweiten Nachweisschwelle einen Nachweissignal-Ausgang (8a, 9a, 10a, 11a) zum Ausgeben von ersten und zweiten Ausgangssignalen aus der ersten und zweiten Eingangsstufe als Ergebnis des jeweiligen Vergleichs aufweisen und wobei den Eingangsstufen (8 bis 11 100A, 100B) mindestens eine Auswertungseinheit (12 bis 16 103) mit Steuersignalausgangen (16a, 16b) zur Ausgabe des die jeweiligen Auswertungsergebnisse charakterisierenden Steuersignals zugeordnet ist, wobei ferner die zweite Eingangsstufe (9, 10; 100B) einen Breitbandverstärker (9.2, 10.2; 104) mit hohem Verstärkungsfaktor sowie eine Pegelbegrenzerschaltung (9.1, 10.1; 104) zur Abschneidung von oberhalb einer vorbestimmten, oberhalb des Pegels der zweiten Nachweisschwelle gewählten Grenze (C_{U}, C_{L}) liegenden Signalanteilen aufweist.

2. Vorrichtung nach Anspruch 1, **dadurch gekennzeichnet, daß** der/die Signalaufnehmer eine intrakardial angeordnete Elektrode (1, 2) aufweist bzw. aufweisen, die den zeitlichen Verlauf eines Herzaktionspotentials liefert.

3. Vorrichtung nach einem der Ansprüche 1 oder 2, **dadurch gekennzeichnet, daß** der/die Signalaufnehmer einen intrakardial angeordneten Druckwandler aufweist bzw. aufweisen, der ein intrakardiale zeitliche Druckschwankungen reflektierendes elektrisches Signalspektrum liefert.

4. Vorrichtung nach Anspruch 1, **dadurch gekennzeichnet, daß** die zweite Eingangsstufe (9, 10; 100B) eine Austast- oder Blanking-Schaltung (9.1, 10.1; 104) zur Ausblendung der Signalanteile während einer vorbestimmten Zeitspanne nach dem Auftreten eines oberhalb der vorbestimmten Grenze liegenden Signalanteils aufweist.

5. Vorrichtung nach einem der Ansprüche 1 bis 4, **dadurch gekennzeichnet, daß** die zweite Eingangsstufe (100B) einen A/D-Wandler (107) zur Digitalisierung der aufgenommenen oder der bereits vorverarbeiteten Signale aufweist und die Vergleichereinheit als digitaler Vergleicher (103, 109) ausgeführt ist.

6. Vorrichtung nach Anspruch 1 und Anspruch 5, **dadurch gekennzeichnet, daß** der Eingang des A/D-Wandlers (107) mit dem Ausgang der Pegelbegrenzerschaltung (104) verbunden ist.

7. Vorrichtung nach Anspruch 5 oder 6, **dadurch gekennzeichnet, daß** ein Signalspeicher (110) vorgesehen ist, dessen Dateneingang mit dem Ausgang des A/D-Wandlers (107) und dessen Datenausgang mit einer internen oder externen Auswertungseinheit (103) verbunden werden kann.

8. Vorrichtung nach einem der Ansprüche 1 bis 7, **dadurch gekennzeichnet, daß** der ersten und zweiten Eingangsstufe (100A, 100B) Zeitgebermittel (103) zur Bestimmung von Auswertungszeitintervallen zugeordnet sind, und sie jeweils eine Ratenbestimmungsschaltung (102, 104) zur Bestimmung einer mittleren Rate der über der jeweiligen Nachweisschwelle liegenden Signalanteile aufweisen.

9. Vorrichtung nach einem der Ansprüche 1 bis 8, **dadurch gekennzeichnet**, - daß Mittel (12 bis 16; 103, 109) zur Amplitudendiskriminierung und ggf. Amplituden-Mittelwertbildung sowie Mittel (16; 103) zur Einstellung der zweiten Nachweisschwelle in Abhängigkeit vom Ergebnis einer Auswertung der maximalen oder einer mittleren Amplitude des keiner Abschneidung unterzogenen Anteils des Gesamt-Signalspektrums während einer initialen Meßperiode vorgesehen sind.

10. Vorrichtung nach Anspruch 9, **dadurch gekennzeichnet, daß** die Mittel zur Amplitudendiskriminierung (103, 109) dem A/D-Wandler (107) nachgeschaltet sind.

11. Vorrichtung nach einem der Ansprüche 1 bis 10, **dadurch gekennzeichnet, daß** die Lese- und Auswertungsschaltung (6; 100) einen Mikroprozessor (16; 103) und/oder eine Auswertungsschaltung vom Kundenwunsch-Typ (109) aufweist.

12. Kombinierter Herzschrittmacher/Defibrillator mit einer Vorrichtung nach einem der Ansprüche 1 bis 11, **dadurch gekennzeichnet, daß** ein Ausgang (16b) der Lese- und Auswertungsschaltung (6) mit einem Steuereingang (5a) einer Entladungsstufe (5) verbunden ist, derart, daß ein das Auftreten von Fibrillationen kennzeichnendes Steuersignal der Entladungsstufe (5) zugeführt wird, und - wahlweise in Abhängigkeit von der Zuführung weiterer Steuersignale - die Abgabe eines Defibrillationsimpulses auslöst. und
daß ein weiterer Ausgang (16a) der Lese- und Auswertungsschaltung (6) mit einem Steuereingang (4a) einer Schrittmacher-Impulserzeugungsstufe (4) verbunden ist, derart, daß ein das Auftreten einer Bradykardie kennzeichnendes Steuersignal der Schrittmacher-Impulserzeugungsstufe zugeführt wird, und - wahlweise in Abhängigkeit von der Zuführung weiterer Steuersignale - die Abgabe eines Stimulationsimpulses auslöst.

## Claims

1. Device for producing control signals for a combined cardiac pacemaker/defibrillator by recording signals characteristic of cardiac actions in the atrium (A) and/or ventricle (V) of a heart (H)
and evaluation thereof
for obtaining information in regard to the occurrence of fibrillations or cardiac actions based on a normal sinus rhythm,
comprising
at least one intracardial signal sensor (1, 2) in the atrium (A) and/or ventricle (V) for recording a signal spectrum in dependence on time,
a reading and evaluation circuit (6; 10) with a threshold characteristic, connected to the signal sensor for feeding the signal spectrum recorded by way of each signal sensor (1, 2),
wherein in the reading and evaluation circuit (6; 100) each signal sensor (1, 2) is connected for feeding the respective signal spectrum and processing thereof including
a first input stage (8, 11) with a first reading amplifier (8.1, 11.1) with a first gain factor (VS(A); VS(V)) which is adjustable but which is constant in respect of time after adjustment has been effected, and
with a first threshold detector circuit (8.4, 11.4) with a first detection threshold (TS(A), TS(V)) which is adjustable but which is constant in respect of time after an adjustment has been effected,
wherein the first reading amplifier (8.1, 11.1) determines the gain factor with which the recorded signal spectrum is amplified as far as the input of the first threshold value detector circuit and the first gain factor (VS(A), VS(V)) together with the first detection threshold (TS(A), TS(V)) is tuned to the signal amplitude of cardiac signals which occur with a normal sinus rhythm, and
a second input stage (9, 10) with a second reading amplifier (9.2, 10.2) with a second gain factor (VL(A); VL(V)) which is adjustable but which is constant in respect of time after adjustment has been effected, and
with a second threshold detector circuit (9.4, 10.4) with a second detection threshold (TL(A), TL(V)) which is adjustable but which is constant in respect of time after an adjustment has been effected,
wherein the second reading amplifier (9.2, 10.2) determines the gain factor with which the recorded signal spectrum is amplified as far as the input of the second threshold value detector circuit and the second gain factor (VL(A), VL(V)) together with the second detection threshold (TL(A), TL(V)) is tuned to the signal amplitude of fibrillation events,
wherein the threshold value detector circuits (8.4, 9.4, 10.4, 11.4) respectively have for separate comparison of the signals of the signal spectrum to the first and second detection threshold a detection signal output (8a, 9a, 10a, 11a) for outputting first and second output signals from the first and second input stage as the result of the respective comparison, and wherein associated with the input stages (8 to 11, 100A, 100B) is at least one evaluation unit (12 to 16, 103) with control signal outputs (16a, 16b) for output of the control signal characterising the respective evaluation results, wherein moreover the second input stage (9, 10; 100B) has a wide-band amplifier (9.2, 10.2; 104) with a high gain factor, and a level limiter circuit (9.1, 10.1; 104) for cutting off signal components which are above a predetermined limit (C_{U}, C_{L}) which is selected above the level of the second detection threshold.

2. Device according to claim 1 **characterised in that** the signal sensor/sensors has or have an intracardially arranged electrode (1, 2) which supplies the configuration in respect of time of a cardiac action potential.

3. Device according to one of claims 1 and 2 **characterised in that** the signal sensor or sensors has or have an intracardially arranged pressure transducer which supplies an electrical signal spectrum reflecting intracardial pressure fluctuations in respect of time.

4. Device according to claim 1 **characterised in that** the second input stage (9, 10; 100B) has a cut-out or blanking circuit (9.1, 10.1; 104) for blanking out the signal components during a predetermined period of time after the occurrence of a signal component which is above the predetermined limit.

5. Device according to one of claims 1 to 4 **characterised in that** the second input stage (100B) has an A/D converter (107) for digitisation of the recorded signals or the signals which have already been pre-processed and the comparator unit is in the form of a digital comparator (103, 109).

6. Device according to claim 1 and claim 5 **characterised in that** the input of the A/D converter (107) is connected to the output of the level limiter circuit (104).

7. Device according to claim 5 or claim 6 **characterised in that** there is provided a signal memory (110) whose data output can be connected to the output of the A/D converter (107) and whose data output can be connected to an internal or external evaluation unit (103).

8. Device according to one of claims 1 to 7 **characterised in that** associated with the first and second input stages (100A, 100B) are timing means (103) for determining evaluation time intervals and they each have a respective rate determining circuit (102, 104) for determining an average rate of the signal components which are above the respective detection threshold.

9. Device according to one of claims 1 to 8 **characterised in that** there are provided means (12 to 16; 103, 109) for amplitude discrimination and possibly amplitude averaging and means (16; 103) for setting the second detection threshold in dependence on the result of evaluation of the maximum or an average amplitude of the component, which is not subjected to a cut-off, of the total signal spectrum during an initial measuring period.

10. Device according to claim 9 **characterised in that** the means for amplitude discrimination (103, 109) are connected downstream of the A/D converter (107).

11. Device according to one of claims 1 to 10 **characterised in that** the reading and evaluation circuit (6; 100) has a microprocessor (16; 103) and/or an evaluation circuit of the customer wish type (109).

12. A combined cardiac pacemaker/defibrillator having a device according to one of claims 1 to 11 **characterised in that** an output (16b) of the reading and evaluation circuit (6) is connected to a control input (5a) of a discharge stage (5) in such a way that a control signal characterising the occurrence of fibrillations is fed to the discharge stage (5) and - selectively in dependence on the feed of further control signals - triggers the delivery of a defibrillation pulse, and a further output (16a) of the reading and evaluation circuit (6) is connected to a control input (4a) of a pacemaker pulse-generating stage (4) in such a way that a control signal characterising the occurrence of a bradycardia is fed to the pacemaker pulse-generating stage and - selectively in dependence on the feed of further control signals - triggers the delivery of a stimulation pulse.

## Revendications

1. Ensemble combiné stimulateur cardiaque/défibrillateur pour l'enregistrement de signaux, caractéristiques pour des actions cardiaques, dans l'oreillette (A) et/ou dans le ventricule (V) d'un coeur (H) et leur évaluation et pour l'obtention d'une indication concernant l'apparition d'actions cardiaques, basées sur un rythme sinusal normal, ou de fibrillations, comportant
respectivement au moins un enregistreur intracardiaque de signaux (1,2) dans l'oreillette (A) et/ou dans le ventricule (V) pour l'enregistrement d'un spectre de signaux en fonction du temps,
un dispositif de lecture et d'évaluation (6;100) présentant une caractéristique de valeur de seuil et relié à l'enregistreur de signaux pour l'envoi du spectre de signaux enregistrés au moyen de chaque enregistreur de signaux (1,2),
dans lequel chaque enregistreur de signaux (1,2) pour l'envoi du spectre respectif de signaux et sa préparation est relié au circuit de lecture et d'évaluation (6;100), l'enregistreur comprenant
un premier amplificateur de lecture (8.1, 11.1) comportant un facteur d'amplification (VS(A); VS(V)) réglable, mais constant dans le temps après un réglage réalisé,
ainsi qu'un premier circuit de détection à valeur de seuil (8.4,11.4) comportant un seuil de détection (TS(A), TS(V)) réglable, mais constant dans le temps après un réglage exécuté,
le premier amplificateur de lecture (8.1, 11.1) déterminant le facteur d'amplification, avec lequel le spectre enregistré du signal jusqu'à l'entrée du premier circuit de détection à valeur de seuil, et le premier facteur d'amplification (VS(A), VS(V)) conjointement avec le premier seuil de détection (TS(A), TS(V)) est réglé sur l'amplitude de signaux cardiaques qui s'établissent dans le cas du rythme sinusal normal, et
un second étage d'entrée (9,10) comportant un second amplificateur de lecture (9.2, 10.2) possédant un second facteur d'amplification (VL(A), VL(V)) réglable, mais constant dans le temps après un réglage exécuté,
ainsi qu'un second circuit de détection à valeur de seuil (9.4, 10.4) comportant un second seuil de détection (TL(A), TL(B)) réglable, mais constant dans le temps après un réglage exécuté,
le second amplificateur de lecture (9.2, 10.2) déterminant le facteur d'amplification, avec lequel le spectre enregistré des signaux est amplifié jusqu'à l'entrée du second circuit de détection à valeur de seuil, et le second facteur d'amplification (VS(A), VS(V)) conjointement avec le second seuil de détection (TL(A), TL(V), est réglé sur l'amplitude des signaux de phénomènes de fibrillation,
dans lequel les circuits de détection à valeur de seuil (8.4, 9.4, 10.4, 11.4) comportent respectivement pour une comparaison séparée des signaux du spectre de signaux aux premier et second seuils de détection, une sortie de signaux de détection (8a,9a,10a,11a) pour la délivrance de premier et second signaux de sortie à partir des premier et second étages d'entrée en tant que résultat de la comparaison, et dans lequel aux étages d'entrée (8 à 11, 100A, 100B) est associée au moins une unité d'évaluation (12 à 18, 130) comportant des sorties (16a, 16B) du signal de commande pour la délivrance du signal de commande caractérisant les résultats d'évaluation respectifs, et dans lequel en outre le second étage d'entrée (9,10;100B) est un amplificateur à large bande (9.2,10.2;104) ayant un facteur d'amplification élevé ainsi qu'un circuit de limitation de niveau (9.1,10.1,104) pour tronquer des composantes de signaux situées au-dessus du niveau d'une limite prédéterminée (C_{U}, C_{L}) choisie au-dessus du niveau du second seuil de détection.

2. Dispositif selon la revendication 1, **caractérisé en ce que** le/les enregistreurs de signaux possèdent une électrode (1,2) disposée de façon intracardiaque et qui fournit la variation dans le temps d'un potentiel d'action cardiaque.

3. Dispositif selon l'une des revendications 1 ou 2, **caractérisé en ce que** le/les enregistreurs de signaux possèdent un transducteur de pression disposé de façon intracardiaque et qui fournit un spectre de signaux électriques reproduisant des variations temporelles intracardiaques de pression.

4. Dispositif selon la revendication 1, **caractérisé en ce que** le second étage d'entrée (9,10;100B) comporte un circuit d'extinction ou de suppression (9.1,10.1;104) pour occulter les composantes de signaux pendant un intervalle de temps prédéterminé après l'apparition d'une composante de signal située au-dessus de la limite prédéterminée.

5. Dispositif selon l'une des revendications 1 à 4, **caractérisé en ce que** le second étage d'entrée (5B) comporte un convertisseur analogique/numérique (107) pour la numérisation des signaux enregistrés ou des signaux déjà prétraités et l'unité de comparaison est réalisée sous la forme d'un comparateur numérique (103,109).

6. Dispositif selon la revendication 1 et la revendication 5, **caractérisé en ce que** l'entrée du convertisseur analogique/numérique (107) est reliée à la sortie du circuit de limitation de niveau (104).

7. Dispositif selon la revendication 5 ou 6, **caractérisé en ce qu'**il est prévu une mémoire de signaux (110), dont l'entrée des données peut être reliée à la sortie du convertisseur analogique/numérique (116) et dont la sortie des données peut être reliée à une unité d'évaluation interne ou externe (130).

8. Dispositif selon l'une des revendications 1 à 7, **caractérisé en ce que** des moyens formant minuterie (103) pour déterminer des intervalles de temps d'évaluation sont associés aux premier et second étages d'entrée (100A, 100B) et que ces moyens comportent respectivement un circuit de détermination de rythme (102,104) pour déterminer un rythme moyen des composantes de signaux qui sont situées au-dessus du seuil respectif de détection.

9. Dispositif selon l'une des revendications 1 à 8, **caractérisé en ce qu'**il est prévu des moyens (12 à 16; 103,109) pour discriminer l'amplitude et éventuellement former la valeur moyenne de l'amplitude ainsi que des moyens (16;103) pour régler le second cycle de détection en fonction du résultat d'une évaluation de l'amplitude maximale ou de l'amplitude moyenne de la composante, qui n'est soumise à aucun écrêtage, de l'ensemble du spectre de signaux pendant une période de mesure initiale.

10. Dispositif selon la revendication 9, **caractérisé en ce que** les moyens de discrimination d'amplitude (103,109) sont branchés en aval du convertisseur analogique/numérique (107).

11. Dispositif selon l'une des revendications 1 à 10, **caractérisé en ce que** le circuit de lecture et d'évaluation (6;100) comporte un microprocesseur (16;103) et/ou un circuit (109) d'évaluation du type de souhait du client.

12. Ensemble combiné stimulateur cardiaque/défibrillateur comportant un dispositif selon l'une des revendications 1 à 11, **caractérisé en ce qu'**une sortie (16b) du circuit de lecture et d'évaluation (6) est reliée à une entrée de commande (5a) d'un étage de décharge (5) de telle sorte qu'un signal de commande caractérisant l'apparition de fibrillations est envoyé à l'étage de décharge (5) et déclenche la délivrance d'une impulsion de défibrillation - au choix en fonction de l'envoi d'autres signaux de commande -, et qu'une autre sortie (16a) du circuit de lecture et d'évaluation (6) est reliée à une entrée de commande (4a) d'un étage (4) de production d'impulsions du stimulateur, de telle sorte qu'un signal de commande, qui caractérise l'apparition d'une bradycardie, est envoyé à l'étage de production d'impulsions du stimulateur, et déclenche la délivrance d'une impulsion de stimulation - au choix en fonction de l'envoi d'autres signaux de commande.
